# FASCICULE DE BREVET EUROPEEN

(11) **EP 1 567 578 B1**
(45) Date de publication et mention de la délivrance du brevet: **25.06.2008**
(21) Numéro de dépôt: 03789473.0
(22) Date de dépôt: 24.11.2003
(51) Int. Cl.: C08G 69/10, C08G 69/48, A61K 47/48, A61K 9/50

(54) **POLYAMINOACIDES FONCTIONNALISES PAR AU MOINS UN GROUPEMENT (OLIGO)AMINOACIDE ET LEURS APPLICATIONS NOTAMMENT THERAPEUTIQUES**
POLYAMINOSÄUREN, FUNKTIONALISIERT DURCH MINDESTENS EINE (OLIGO-)AMINOSÄURE UND IHRE, INSBESONDERE THERAPEUTISCHE, ANWENDUNG
POLYAMINO ACIDS FUNCTIONALIZED BY AT LEAST ONE (OLIGO)AMINO ACID GROUP AND THERAPEUTIC USES

(30) Priorité: 04.12.2002 FR 0215269
(43) Date de publication de la demande: 31.08.2005
(73) Titulaire: FLAMEL TECHNOLOGIES, 69200 Vénissieux (FR)
(72) Inventeur: ANGOT, Stéphanie, 33200 BORDEAUX (FR); CHAN, You-Ping, F-69008 Lyon (FR); SOULA, Gérard, F-69330 Meyzieu (FR)
(74) Mandataire: Fleurance, Raphaël
(86) Numéro de dépôt international: PCT/FR2003/003458
(87) Numéro de publication internationale: WO 2004/060968

(56) Documents cités:
- EP-A- 0 734 720
- WO-A-00/30618
- WO-A-87/03891
- HEESWIJK VAN W A R ET AL: "THE SYNTHESIS AND CHARACTERIZATION OF POLYPEPTIDE-ADRIAMYCIN CONJUGATES AND ITS COMPLEXES WITH ADRIAMYCIN. PART I" JOURNAL OF CONTROLLED RELEASE, ELSEVIER SCIENCE PUBLISHERS B.V. AMSTERDAM, NL, vol. 1, 1985, pages 301-315, XP002059418 ISSN: 0168-3659 cité dans la demande
- GONSALVES K E ET AL: "Synthesis and Properties of Degradable Polyamides and Related Polymers" TRENDS IN POLYMER SCIENCE, ELSEVIER SCIENCE PUBLISHERS B.V. AMSTERDAM, NL, vol. 4, no. 1, 1996, pages 25-31, XP004049307 ISSN: 0966-4793

## Description

La présente invention concerne des nouveaux matériaux à base de polyaminoacides biodégradables, utiles notamment pour la vectorisation de principe(s) actif(s) (PA).

L'invention vise aussi de nouvelles compositions pharmaceutiques, cosmétiques, diététiques ou phytosanitaires à base de ces polyaminoacides. Ces compositions peuvent être du type de celles permettant la vectorisation de PA et se présentant de préférence sous forme d'émulsions, de micelles, de particules, de gels, d'implants ou de films.

Les PA considérés sont, avantageusement, des composés biologiquement actifs et qui peuvent être administrés à un organisme animal ou humain par voie orale, parentérale, nasale, vaginale, oculaire, sous-cutanée, intraveineuse, intra-musculaire, intradermique, intrapéritonéale, intracérébrale, buccale, etc.

Les PA plus particulièrement mais non limitativement concernés par l'invention sont des protéines, des glycoprotéines, des peptides, des polysaccharides, des lipopolysaccharides, des oligo ou des polynucléotides, et des molécules organiques. Mais il peut aussi s'agir de produits cosmétiques ou de produits phytosanitaires, tels que des herbicides, des insecticides, des fongicides, etc.

Dans le domaine de la vectorisation des principes actifs notamment médicamenteux, il existe un besoin, dans beaucoup de cas :
- de les protéger contre la dégradation (hydrolyse, précipitation sur site, digestion enzymatique etc..) jusqu'à ce qu'ils atteignent leur site d'action,
- et/ou de contrôler leur vitesse de libération afin de maintenir un niveau thérapeutique sur une durée définie,
- et/ou de les véhiculer (en les protégeant) au site d'action.

A ces fins, plusieurs types de polymères ont été étudiés et certains sont même disponibles commercialement. On peut citer par exemple les polymères du type polylactique, polylactique-glycolique, polyoxyethylène-oxypropylène, polyaminoacide ou encore polysaccharide. Ces polymères constituent des matières premières permettant de fabriquer, par exemple, des implants massiques, des microparticules, des nanoparticules, des vésicules, des micelles ou des gels. Outre le fait que ces polymères doivent être adaptés à la fabrication de tels systèmes, ils doivent également être biocompatibles, non-toxiques, non-immunogènes, économiques et ils doivent pouvoir être facilement éliminés du corps et/ou biodégradables. Sur ce dernier aspect, il est de surcroît essentiel que la biodégradation dans l'organisme génère des produits non-toxiques.

A titre d'illustration de l'art antérieur concernant des polymères employés comme matières premières pour la réalisation de systèmes de vectorisation de PA, divers brevets ou demandes de brevet ou articles scientifiques sont évoqués ci-après.

Le brevet US-B-4 652 441 décrit des microcapsules de polylactide encapsulant l'hormone LH-RH. Ces micro capsules sont produites en préparant une émulsion eau-dans-huile-dans-eau et comprennent une couche interne aqueuse contenant l'hormone, une substance (gélatine) fixant cette dernière, une couche huileuse de polylactide, ainsi qu'une couche externe aqueuse (alcool polyvinylique). La libération du PA peut se faire sur une période de plus de deux semaines après injection sous-cutanée.

Le brevet US-B-6153 193 décrit des compositions à base de micelles de poly(oxyéthylène)-poly(oxypropylène) amphiphiles, pour la vectorisation d'anti-cancéreux tel que l'adriamycine.

Akiyoshi et al. (J. Controlled Release 1998, 54, 313-320) décrivent des pullulans qui sont rendus hydrophobes par greffage de cholestérol et qui forment des nanoparticules dans l'eau. Ces nanoparticules aptes à se complexer de manière réversible avec l'insuline, forment des suspensions colloïdales stables.

Le brevet US-B-4 351 337 décrit des copolyaminoacides amphiphiles, à base de leucine et de glutamate, utilisables sous forme d'implants ou de microparticules pour la libération contrôlée de principes actifs. La libération de ces derniers peut se faire sur une durée très longue dépendant de la vitesse de dégradation du polymère.

Le brevet US-B-4 888 398 décrit des polymères à base de polyglutamate ou polyaspartate, et éventuellement polyleucine, avec des groupements pendants de type allcyloxycarbonylinéthyle, placés de façon aléatoire sur la chaîne polyaminoacide. Ces polyaminoacides, greffés par des groupements latéraux e.g. méthoxycarbonylméthyle, sont utilisables sous forme d'implants biodégradables contenant un PA à libération prolongée.

Le brevet US-B-5 904 936 décrit des nanoparticules obtenues à partir d'un polymère bloc polyleucine-polyglutamate, aptes à former des suspensions colloïdales stables et capables de s'associer spontanément avec des protéines biologiquement actives sans les dénaturer. Ces dernières peuvent ensuite être libérées in vivo de manière contrôlée, sur une longue période.

Le brevet US-B-5 449 513 décrit des copolymères bloc amphiphiles comprenant un bloc polyoxyéthylène et un bloc polyaminoacide, par exemple poly(bêta-benzyl-L-aspartate). Ces polymères polyoxyéthylène-polybenzylaspartate forment des micelles qui sont aptes à encapsuler des molécules actives hydrophobes telles que adriamycine ou l'indométhacine.

La demande de brevet WO-A-99/61512 décrit des polylysines et des polyornithines fonctionnalisées par un groupe hydrophobe (acide palmitique relié à la polylysine ou ornithine) et un groupe hydrophile (polyoxyéthylène). Ces polymères, par exemple la polylysine greffée avec des chaînes polyoxyéthylène et palmitoyle forment en présence de cholestérol des vésicules capables d'encapsuler la doxorubicine ou l'ADN. Ces polymères à base de polylysines sont cationiques en milieu physiologique.

La demande de brevet WO-A-02/28251 de la demanderesse concerne une suspension de particules biocompatibles de vectorisation (PV) de principes actifs (PA). Ces PV sont à base d'un copolymère dibloc *polyaminoacide neutre hydrophile* poly(AANI) / *polyaminoacide neutre hydrophobe* poly(AANO). Ces particules de polyAANI/polyAANO sont aptes à associer en suspension colloïdale à l'état non dissous, au moins un PA et à libérer celui-ci, notamment in vivo, de manière prolongée et/ou retardée. Ces nouvelles **PV** forment spontanément et sans l'aide de tensioactifs ou de solvants organiques, des suspensions aqueuses stables. Le copolymère dibloc *polyaminoacide neutre. hydrophile* poly(AANI) / *polyaminoacide neutre hydrophobe* (polyAANO) peut être par exemple : poly(Gln-N-hydroxyéthyle) / poly(Leu), issu de l'aminolyse de poly(Glu-O-alkyle) / poly(Leu) par de l'hydroxyéthylamine.

Ces copolymères sont neutres en milieu physiologique.

La demande de brevet WO-A-00/30618, de la demanderesse, décrit des polymères blocs ou aléatoires poly(glutamate de sodium)-poly(glutamate de méthyle, d'éthyle, d'hexa-décyle ou de dodécyle), aptes à former des suspensions colloïdales stables et capables de s'associer spontanément avec des protéines biologiquement actives sans les dénaturer. Ces dernières peuvent ensuite être libérées in vivo de manière contrôlée, sur une longue période. Ces copolyaminoacides amphiphiles sont modifiés par la présence d'une chaîne latérale alkyle hydrophobe. Ces groupements alkyles sont greffés de façon covalente sur le polymère via une fonction ester. Ces polymères sont anioniques en milieu physiologique.
Ils restent perfectibles à au moins deux égards, selon l'application visée :
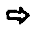 la stabilité relative de la fonction ester dans un milieu aqueux,
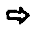 et la mise en oeuvre comme précurseurs de greffons alkyles hydrophobes, de certains alcools non naturels tels que l'hexanol. Ce dernier aspect est notamment problématique en terme de toxicité, si la concentration en polymère chargé par ces alcools résiduels devient importante.

En ce qui concerne l'état de l'art sur les polyaminoacides branchés, décrits dans la littérature et qui sont fonctionnalisés par des oligoaminoacides, on note les travaux suivants :
Le brevet WO-A-87/03891 décrit des polyglutamates ou polyaspartates porteurs de groupements diacides de type malonique ou succinique, liés à la chaîne polyaminoacide via une rotule d'espacement ("espaceur") de nature oligopeptidique. La présence du groupement diacide permet de fixer des cations calcium ou de former des anhydrides cycliques susceptibles de réagir avec un principe actif. Ces polymères sont utilisables notamment sous forme d'implants pour la libération lente in vivo d'un principe actif. Dans le même esprit, Hoes et al. [J. Controlled Release 1 (1985) 301-315 & 2 (1985), 205-213] décrivent des polyglutamates dans lesquels un composé actif anticancéreux (l'adriamycine) est greffé au polymère via une rotule d'espacement "espaceur" glycine-glycine-leucine, facilement dégradée in vivo.
Dans un autre contexte, des polyaminoacides branchés à base de polylysine ont été synthétisés pour leur évaluation en immunologie (Hudecz et al. Polymeric Materials in Medication. Plenum Press, New York, 1985, pages 265-289) ou pour des études physiques *(*Mezo et al. J. Controlled Release 2000, 63, 81-95). Ces polymères ont un squelette en polylysine et chaque motif lysine est branché à un oligopeptide hydrophile.
L'utilisation de ces polymères pour associer et/ou vectoriser des principes actifs, non liés aux polymères, n'est pas enseignée par ce document.

Ainsi, même s'il existe de très nombreuses solutions techniques dans l'art antérieur, développées et proposées pour la vectorisation des principes actifs médicamenteux, la réponse à l'ensemble des exigences est difficile à obtenir et demeure non satisfaisante. Plus spécifiquement, la conception d'un polyaminoacide greffé par des (oligo)aminoacides, capable de former une suspension aqueuse colloïdale stable de particules de vectorisation propres à s'associer réversiblement à des principes actifs, n'est pas décrite à ce jour.

Dans ce contexte, l'un des objectifs essentiels de la présente invention est de fournir une nouvelle famille de polymères anioniques à pH physiologique animal (par exemple de l'ordre de 7,4), à base de polyglutamate et polyaspartate, qui représentent un perfectionnement par rapport à ceux décrits dans la demande de brevet WO-A-00/30618, notamment en termes de stabilité et de non toxicité.

Un autre objectif essentiel de la présente invention est que ces polymères soient aptes à être utilisés pour la vectorisation de PA et permettent de satisfaire de manière optimale à toutes les spécifications du cahier des charges, à savoir notamment :
o capacité :
   ■ à former aisément et économiquement des suspensions colloïdales aqueuses stables,
   ■ à s'associer facilement avec de nombreux principes actifs,
   ■ et à libérer ces principes actifs in vivo,
o biocompatibilité,
o biodégradabilité,
o stabilité à l'hydrolyse.

Cet objectif, parmi d'autres, est atteint par la présente invention qui concerne tout d'abord un polyaminoacide comprenant des unités aspartiques et/ou des unités glutamiques, dont certaines sont porteuses d'au moins un greffon, caractérisé :
- en ce qu'au moins un de ces greffons est relié à une unité aspartique ou glutamique, par l'intermédiaire d'une liaison amide,
- en ce qu'au moins une partie de ces greffons comprend un ou plusieurs (oligo)aminoacide(s), à l'exclusion des greffons porteurs d'au moins un diacide carboxylique cyclisable en anhydride,
- et en ce que l'unité ou les unités "acides aminés" comprises dans l'(oligo)aminoacide sont choisies parmi celles ayant un groupement alkyle ou aryle en alpha, de préférence parmi celles comprises dans le groupe comportant l'alanine, la valine, la leucine, l'isoleucine et la phénylalanine.

Il est du mérite de la demanderesse d'avoir eu l'idée de combiner, de façon tout à fait judicieuse et avantageuse, des polyaminoacides particuliers polyAsp et/ou polyGlu, biodégradables et anioniques avec des greffons comportant au moins un motif (oligo)aminoacide et reliés au squelette polyAsp et/ou polyGlu par une liaison amide.

Ces nouveaux (co)polymères se sont avérés être particulièrement bien adaptés pour la vectorisation des protéines.

Conformément à une forme préférée de réalisation de l'invention, chaque greffon est relié à une unité aspartique ou glutamique par l'intermédiaire d'une liaison amide.

Au sens de l'invention le terme *« polyaminoacide »* couvre aussi bien les oligoaminoacides comprenant de 2 à 20 unités "acide aminé" que les polyaminoacides comprenant plus de 20 unités "acide aminé".

De préférence, l'oligoaminoacide ou les (oligo)aminoacides de tout ou partie des greffons est (sont) constitué(s) (chacun) par des unités "acide aminé" identiques entre elles.

Suivant une caractéristique préférée de l'invention, le nombre d'unités "acide aminé" par greffon varie de 1 à 6 unités.

Il va de soi que conformément à l'invention, les unités "acide aminé" constitutives des greffons peuvent être identiques ou différentes entre elles.

Ces polymères présentent des propriétés surprenantes d'association et/ou d'encapsulation avec un ou plusieurs principes actifs, en comparaison avec des produits analogues. De plus, ils sont facilement dégradés, en présence d'enzymes, en catabolites/métabolites non toxiques (acides aminés).
Au sens de l'invention et dans tout le présent exposé, les termes "association" ou "associer" employés pour qualifier les relations entre un ou plusieurs principes actifs et les polyaminoacides, signifient en particulier que le ou les principes actifs sont liés au(x) polyaminoacide(s) notamment par une liaison faible, par exemple par liaison ionique et/ou par contact hydrophobe, et/ou sont encapsulés par le ou les polyaminoacides.

De préférence, les polyaminoacides selon la présente invention sont des oligomères ou des homopolymères comprenant des unités récurrentes acide glutamique ou aspartique ou des copolymères comprenant un mélange de ces deux types d'unités "acide aminé". Les unités considérées dans ces polymères sont des acides aminés ayant la configuration D ou L ou D/L et sont liées par leurs positions alpha ou gamma pour l'unité glutamate ou glutamique et alpha ou bêta pour l'unité aspartique ou aspartate.

Les unités "acide aminé" préférées de la chaîne polyaminoacide principale sont celles ayant la configuration L et une liaison de type alpha.

De manière plus préférée encore, les polyaminoacides selon l'invention répondent à la formule générale (I) suivante : 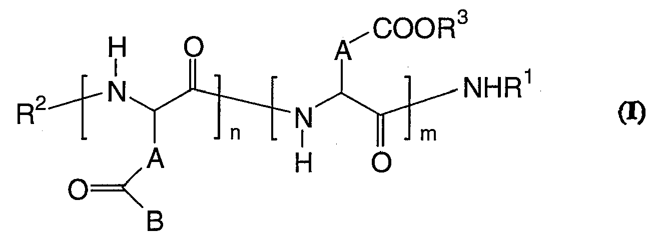 dans laquelle :
■ R¹ représente un H, un alkyle linéaire en C2 à C 10 ou ramifié en C3 à C10, un benzyle, une unité "acide aminé" terminale ;
■ R² représente un H, un groupe acyle linéaire en C2 à C10 ou ramifié en C3 à C10, ou un pyroglutamate ;
■ R³ est un H ou une entité cationique, de préférence sélectionnée dans le groupe comprenant :
   - les cations métalliques avantageusement choisis dans le sous-groupe comprenant : le sodium, le potassium, le calcium, le magnésium ;
   - les cations organiques avantageusement choisis dans le sous-groupe comprenant :
      - les cations à base d'amine,
      - les cations à base d'oligoamine,
      - les cations à base de polyamine (la polyéthylèneimine étant particulièrement préférée),
      - les cations à base d'acide(s) aminé(s) avantageusement choisis dans la classe comprenant les cations à base de lysine ou d'arginine,
   - ou les polyaminoacides cationiques avantageusement choisis dans le sous-groupe comprenant la polylysine ou l'oligolysine;
■ les n groupements B représentent chacun indépendamment les uns des autres un radical monovalent de formule suivante : 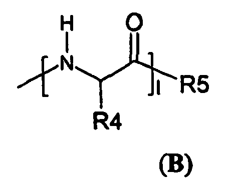
dans laquelle :
- R⁴ représente un méthyle(alanine), isopropyle (valine), isobutyle (leucine), secbutyle (isoleucine), benzyle (phénylalanine), les acides aminés mentionnés entre parenthèses sont ceux qui correspondent à l'unité "acide aminé" formée lorsque R⁴ représente l'alkyle considéré ;
- R⁵ représente un groupement choisi parmi OH, NH₂, un groupement alkoxy en C1 à C5, un benzyloxy ;
   ■ A représente indépendamment un -CH₂- (unité aspartique) ou -CH₂-CH₂-(unité glutamique) ;
   ■ n/(n+m) est défini comme le taux de greffage molaire et varie de 0,5 à 100 % molaire;
   ■ n + m varie de 3 à 1000, de préférence entre 30 et 300 ;
   ■ 1 varie de 1 à 6.

Avantageusement, la longueur de la chaîne (β) des greffons déterminée par la valeur de 1, d'une part, et le choix du motif alkyle R⁴, d'autre part, permettent de régler la balance hydrophile/hydrophobe du polymère selon l'application visée.

Selon un premier mode de réalisation de l'invention, les polyaminoacides sont des homopolymères d'alpha-L-glutamate ou d'alpha-L-glutamique.

Selon un deuxième mode de réalisation de l'invention, les polyaminoacides sont des homopolymères d'alpha-L-aspartate ou d'alpha-L-aspartique.

Selon un troisième mode de réalisation de l'invention, les polyaminoacides sont des copolymères d'alpha-L-aspartate/alpha-L-glutamate ou d'alpha-L-aspartique/alpha-L-glutamique.

Avantageusement, la distribution des unités aspartiques et/ou glutamiques de la chaîne polyaminoacide principale est telle que les polymères ainsi constitués sont soit aléatoires, soit de type bloc, soit de type multibloc.

Selon un autre mode de définition, les polyaminoacides selon l'invention ont une masse molaire qui se situe entre 2 000 et 100 000 g/mole, et de préférence entre 5 000 et 40 000 g/mole.

Il est par ailleurs préférable que le taux de greffage molaire en motif (oligo)aminoacide des polyaminoacides selon l'invention, soit compris entre 2 et 70 %, et de préférence entre 5 et 40 %.

De manière remarquable, les polyaminoacides de l'invention sont susceptibles d'être utilisés de plusieurs façons selon le taux de greffage. Les méthodes de mise en forme d'un polymère pour l'encapsulation d'un principe actif sous les diverses formes visées par l'invention sont connues de l'homme de l'art. Pour plus de détails, on peut se référer, par exemple à ces quelques références particulièrement pertinentes :
"Microspheres, Microcapsules and Liposomes ; vol 1. Preparation and chemical applications" Ed. R. Arshady, Citus Books 1999. ISBN : 0-9532187-1-6.
"Sustained-Release Injectable Products" Ed. J. Senior et M. Radomsky, Interpharm Press 2000. ISBN : 1-57491-101-5.
"Colloidal Drug Delivery Systems" Ed. J. Kreuter, Marcel Dekker, Inc. 1994. ISBN : 0-8247-9214-9.
"Handbook of Pharmaceutical Controlled Release Technology" Ed. D.L. Wise, Marcel Dekker, Inc. 2000. ISBN : 0-8247-0369-3.

Les polyaminoacides sont en outre extrêmement intéressants, du fait qu'à un taux de greffage relativement faible de l'ordre de 3 à 30 % (variable selon l'(oligo)aminoacide choisi), ils se dispersent dans l'eau à pH 7,4 (par exemple avec un tampon phosphate) pour donner des solutions ou des suspensions colloïdales ou des gels, en fonction de la concentration de polymères et du taux de greffage. De plus, les polyaminoacides (sous forme de particules ou non), peuvent encapsuler ou s'associer aisément avec des principes actifs tels que des protéines, peptides ou petites molécules. La mise en forme préférée est celle décrite dans la demande de brevet WO-A-00/30618 de la demanderesse et qui consiste à disperser le polymère dans l'eau et à incuber la solution en présence d'un PA. Cette solution colloïdale de particules de vectorisation constituées des polyaminoacides selon l'invention, peut ensuite être filtrée sous 0,2 µm puis directement injectée à un patient.

Cette forme particulaire selon la demande de brevet WO-A-00/30618 est notamment envisageable en l'espèce, au-delà de 30 % de taux de greffage et selon l'(oligo)peptide choisi. Le polymère peut alors former des microparticules capables d'associer ou d'encapsuler des PA. Dans ce contexte, la mise en forme des microparticules peut se faire en co-solubilisant le PA et le polymère dans un solvant organique approprié puis en précipitant le mélange dans l'eau. Les particules sont ensuite récupérées par filtration et peuvent ensuite être utilisées pour une administration par voie orale (sous forme de gélule, sous forme compactée et/ou enrobée ou bien encore sous forme dispersée dans une huile) ou par voie parentérale après redispersion dans l'eau.

A des taux supérieurs à 50 % de greffage, la redispersion du polymère en phase aqueuse devient plus difficile du fait de la quantité plus faible des fonctions carboxylate ionisables et le polymère précipite. Dans ce cas, le polymère peut être solubilisé dans un solvant biocompatible tel que la N-méthylpyrrolidone ou une huile appropriée telle que le Mygliol® puis injecté en intramusculaire ou sous-cutanée ou dans une tumeur. La diffusion du solvant ou de l'huile conduit à la précipitation du polymère sur le site d'injection et forme ainsi un dépôt Ces dépôts assurent ensuite une libération contrôlée par diffusion et/ou par érosion et/ou par dégradation hydrolytique ou enzymatique du polymère.

Indépendamment du fait que la forme microparticulaire du polyaminoacide selon l'invention est préférée, les polymères de l'invention, sous forme neutre ou ionisée, sont de façon plus générale, utilisables seuls ou dans une composition liquide, solide ou gel et dans un milieu aqueux ou organique.

Il convient de comprendre que le polymère à base de polyaminoacides contient des fonctions carboxyliques qui sont soit neutres (forme COOH), soit ionisées (anion COO⁻), selon le pH et la composition. Pour cette raison, la solubilité dans une phase aqueuse est directement fonction du taux de COOH libre du polymère (non greffé par le motif hydrophobe) et du pH. En solution aqueuse, le contre-cation peut être un cation métallique tel que le sodium, le calcium ou le magnésium, ou un cation organique tel que la triéthanolamine, la tris(hydroxyméthyl)-aminométhane ou une polyamine tel que la polyéthylèneimine.

Les polymères de l'invention sont par exemple obtenus par des méthodes connues de l'homme de l'art. Les polyaminoacides peuvent être obtenus par greffage de l'(oligo)aminoacide directement sur le polymère par une réaction classique de couplage.

On prépare par exemple un polyaminoacide, homopolyglutamate, homopoly-aspartate ou un copolymère glutamate/aspartate, bloc, multibloc ou aléatoire selon des méthodes classiques.

Pour l'obtention de polyaminoacide de type alpha, la technique la plus courante est basée sur la polymérisation d'anhydrides de N-carboxy-aminoacides (NCA), décrites, par exemple, dans l'article "Biopolymers, 1976, 15, 1869 et dans l'ouvrage de H.R. Kricheldorf "alpha-Aminoacid-N-carboxy Anhydrides and related Heterocycles" Springer Verlag (1987). Les dérivés d'NCA sont de préférence des dérivés NCA-O-Me, NCA-O-Et ou NCA-O-Bz (Me = Méthyl, Et = Ethyle et Bz = Benzyle). Les polymères sont ensuite hydrolysés dans des conditions appropriées pour obtenir le polymère sous sa forme acide. Ces méthodes sont inspirées de la description donnée dans le brevet FR-A-2 801 226 de la demanderesse. Un certain nombre de polymères utilisables selon l'invention, par exemple, de type poly(alpha-L-aspartique), poly(alpha-L-glutamique), poly(alpha-D-glutamique) et poly(gamma-L-glutamique) de masses variables sont disponibles commercialement. Le polyaspartique de type alpha-bêta est obtenu par condensation de l'acide aspartique (pour obtenir un polysuccinimide) suivie d'une hydrolyse basique (cf. Tomida et al. Polymer 1997, 38, 4733-36).

Le couplage de l'(oligo)amine avec une fonction acide du polymère est réalisé aisément par réaction du polyaminoacide en présence d'un carbodiimide comme agent de couplage et optionnellement, un catalyseur tel que le 4-dimethylaminopyridine et dans un solvant approprié tel que la diméthylformamide (DMF), la N-méthyl pyrrolidone (NMP) ou la diméthylsulfoxide (DMSO). Le carbodiimide est par exemple, le dicyclohexylcarbodiimide ou le diisopropylcarbodiimide. Le taux de greffage est contrôlé chimiquement par la stoechiométrie des constituants et réactifs ou le temps de réaction. Les (oligo)aminoacides peuvent êtres obtenus par synthèse séquentielle selon des méthodes classiques (voir par exemple l'ouvrage *"*Principles of Peptide Synthesis" par Bodanszky, Springer-Verlag 1984) ou sont disponibles commercialement.

Selon un autre de ses aspects, l'invention vise une composition pharmaceutique, cosmétique, diététique ou phytosanitaire comprenant au moins un polyaminoacide tel que défini ci-dessus et éventuellement au moins un principe actif, qui peut être thérapeutique, cosmétique, diététique ou phytosanitaire.

Selon encore un autre de ses aspects, l'invention vise une composition notamment, pharmaceutique, cosmétique, diététique ou phytosanitaire comprenant au moins un polyaminoacide comprenant des unités aspartiques et/ou des unités glutamiques dont certaines sont porteuses d'au moins un greffon :
- au moins un de ces greffons étant relié à une unité aspartique ou glutamique, par l'intermédiaire d'une liaison amide,
- au moins une partie de ces greffons comprenant un ou plusieurs (oligo)aminoacide(s),
- et les greffons porteurs d'au moins un diacide carboxylique cyclisable en anhydride étant exclus,
caractérisée en ce qu'elle comprend au moins un principe actif associé au(x) polyaminoacide(s) par une ou plusieurs liaisons autre(s) qu'une (ou que des) liaison(s) chimique(s) covalente(s).

De préférence, le principe actif est une protéine, une glycoprotéine, une protéine liée à une ou plusieurs chaînes polyalkylèneglycol (de préférence PolyÉthylèneGlycol (PEG) : "protéine-PEGylée"), un polysaccharide, un liposaccharide, un oligonucléotide, un polynucléotide ou un peptide.

Plus préférentiellement encore, le principe actif est une petite molécule organique hydrophobe, hydrophile ou amphiphile.

Cette composition peut être sous forme de nanoparticules, de microparticules, d'émulsions, de gels, de micelles, d'implants, de poudres ou de films.

Suivant l'une de ses formes particulièrement préférées, la composition, chargée ou non en principe actif(s), est une suspension colloïdale stable de nanoparticules et/ou de microparticules et/ou de micelles de polyaminoacides, dans une phase aqueuse.

La composition selon l'invention, dès lors qu'elle est pharmaceutique, peut être administrée par voie orale, parentérale, nasale, vaginale, oculaire, sous-cutanée, intraveineuse, intramusculaire, intradermique, intrapéritonéale, intracérébrale ou buccale.

Il est également envisageable que la composition soit sous forme de solution dans un solvant biocompatible, susceptible d'être injectée en sous-cutané, intramusculaire ou dans une tumeur.

Selon une autre variante, la composition selon l'invention est formulée de telle sorte qu'elle soit apte à former un dépôt sur le site d'injection.

L'invention vise aussi des compositions qui comprennent des polyaminoacides selon l'invention et des PA et qui sont susceptibles d'être utilisées pour la préparation :
- de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyÉthylèneGlycol (PEG), on parle alors de protéines "PEGylées"}, des peptides, des polysaccharides, des liposaccharides, des oligonucléotides, des polynucléotides et des petites molécules organiques hydrophobes, hydrophiles ou amphiphiles ;
- et/ou des nutriments,
- et/ou de produits cosmétiques ou phytosanitaires.

Cette préparation est caractérisée en ce qu'elle consiste essentiellement à mettre en oeuvre au moins l'un des polyaminoacides selon l'invention, tels qu'ils sont définis ci-dessus, et/ou les compositions également décrites supra.

Les techniques d'association d'un ou de plusieurs PA aux polyaminoacides greffés selon l'invention, sont décrites notamment dans la demande de brevet WO-A-00/30618. Elles consistent à incorporer au moins un principe actif dans le milieu liquide contenant des particules PV, de manière à obtenir une suspension colloïdale de PV chargées en ou associées avec un ou plusieurs principe(s) actif(s) PA. Cette incorporation, qui conduit à un piégeage de PA par les PV, peut être réalisée de la manière suivante :
- mise en solution aqueuse de PA, puis ajout des PV, soit sous forme de suspension colloïdale, soit sous forme de PV isolées (lyophilisat ou précipitat) ;
- ou ajout de PA, soit en solution, soit à l'état pur ou préformulé, à une suspension colloïdale de particules PV, éventuellement préparée extemporanément par la dispersion de PV sèches dans un solvant approprié, tel que l'eau.

L'invention concerne également une méthode de traitement thérapeutique consistant essentiellement à administrer la composition telle que décrite dans le présent exposé, par voie orale, parentérale, nasale, vaginale, oculaire, sous-cutanée, intraveineuse, intramusculaire, intradermique, intrapéritonéale, intracérébrale ou buccale.

L'invention concerne en outre une méthode de traitement thérapeutique consistant essentiellement à mettre en oeuvre une composition telle que décrite supra sous forme de solution dans un solvant biocompatible puis de l'injecter en sous-cutané, intramusculaire ou dans une tumeur, de préférence de manière à ce qu'elle forme un dépôt sur le site d'injection.

Comme exemples de PA susceptibles d'être associés aux polyaminoacides selon l'invention, qu'ils soient ou non sous forme de (nano ou micro)particules, on peut citer :
o les protéines telles que l'insuline, les interférons, les hormones de croissance, les interleukines, l'érythropoïétine ou les cytokines;
o les peptides telles que la leuprolide ou la cyclosporine ;
o les petites molécules telles que celles appartenant à la famille des antbracyclines, des taxoïdes ou des camptothécines ;
o et leurs mélanges.

L'invention sera mieux comprise et ses avantages et variantes de mise en oeuvre ressortiront bien des exemples qui suivent et qui décrivent la synthèse des polyaminoacides greffés par un groupement (oligo)aminoacide, leur transformation en système de vectorisation de PA (suspension colloïdale aqueuse stable) et la démonstration de la capacité d'un tel système de s'associer à des PA (petites molécules organiques, protéines...) pour former des compositions pharmaceutiques.

### Exemple 1 : Préparation du polymère P1

### Synthèse d'un polyglutamate greffé avec un greffon trileucine amide.

1/ Structure du greffon (Leu)₃NH₂ : - RNCAS 73237-77-1 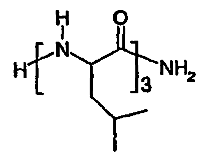 commercialisé par SIGMA
2/ Synthèse du polymère :
   On solubilise 4 g d'un alpha-L-polyglutamate (de masse équivalente à environ 12 000 g/mole par rapport à un standard en polyoxyéthylène et obtenue par polymérisation de monomères constitués par des dérivés N-CarboxyAnhydride de glutamate de méthyle : NCAGluOMe. Cette polymérisation est suivie d'une hydrolyse (comme décrites dans la demande de brevet FR-A-2 801 226) dans 77 ml de DiMéthylFormamide (DMF) en chauffant à 80°C pendant 2 heures. Une fois le polymère solubilisé, on laisse revenir la température à 25 °C et on ajoute successivement 0,99 g du greffon (Leu)₃NH₂ préalablement solubilisé dans 2 ml de DMF, 0,068 g de 4-dimethylaminopyridine préalablement solubilisé dans 1 ml de DMF et 0,43 g de diisopropylcarbodiimide préalablement solubilisé dans 0,5 ml de DMF. Après 8 heures à 25 °C sous agitation, le milieu réactionnel est versé dans 280 ml d'eau contenant 15 % de chlorure de sodium et d'acide chlorhydrique (pH 2). Le polymère précipité est ensuite récupéré par filtration, lavé par de l'acide chlorhydrique 0,1 N puis par du chloroforme. Le polymère est ensuite séché à l'étuve sous vide à 40 °C. On obtient un rendement de l'ordre de 80 %. Le taux de greffage estimé par RMN du proton est d'environ 8 %.

### Exemple 2 : Préparation du polymère P2 à P5

On prépare les polymères P2 à P5 dans les mêmes conditions que celles utilisées pour le polymère P1 en faisant varier le taux de greffage et la nature de l'(oligo)aminoacide.

Les greffons (Val)₃ NH₂ et (Phe)₂ NH₂ sont commercialisés sous forme HCI par la Société BACHEM. Ils sont utilisés après déprotonation par la triéthylamine.

Le greffon (Leu) NH₂ est commercialisé par la Société ALDRICH. Le tableau ci-dessous rassemble les caractéristiques des polymères synthétisés.

**Tableau 1**

| **Polymère** | **Greffon*** | **Taux de greffage (RMN)** | **Mn** g/mole (équiv. PMMA)** |
|---|---|---|---|
| P1 | (Leu)₃NH₂ | 8 % | 19500 |
| P2 | (Leu)₃NH₂ | 21 % | 17300 |
| P3 | (Val)₃NH₂ | 22 % | 18300 |
| P4 | (Phe)₂NH₂ | 22 % | 17300 |
| P5 | (Leu)NH₂ | 40 % | 29300 |

| | | | |
|---|---|---|---|
| * Leu : L-leucine, Val : L-valine, Phe : L-phenylalanine. ** Mn : masse molaire en nombre. | | | |

Dans tous les cas, les polymères sont dispersables dans l'eau à pH 7,4 à environ 20 mg/ml et sont limpides. Une analyse de ces polymères par diffraction de la lumière montre qu'ils forment, selon le taux de greffage et la concentration, des objets de 20 à 200 nm.

### Exemple 3 : Adsorption d'un colorant sur le polymère P1, P3 et P4

Selon l'un des objets de l'invention, les polymères peuvent être utilisés dans l'eau et s'associer avec ou encapsuler un principe actif (sous forme de suspension colloïdale ou non). Pour cette application, il est démontré dans l'expérience ci-après que les polymères P1, P3 et P4 sont capables d'associer ou d'encapsuler un colorant modèle.

L'étude est réalisée de la façon suivante : on solubilise les polymères dans une solution aqueuse à pH 7 (tampon phosphate) et on ajoute 5 mg du colorant dénommé Orange OT (Rn CAS : 2646-17-5). On laisse les solutions dans un bain d'ultrasons pendant une heure pour réaliser l'association. Les solutions sont ensuite centrifugées pour éliminer le colorant non-associé et on mesure la densité optique (DO) au λmax du colorant (495 nm), après dilution. L'essai avec le polyglutamate seul sert de référence.

**Tableau 2**

| **Polymère** | **Concentration polymère** | **DO induite relative** |
|---|---|---|
| P1 | 15,6 mg/ml | 0,27 |
| P3 | 8 mg/ml | 0,27 |
| P4 | 8 mg/ml | 0,21 |
| Polyglutamate | 25 mg/ml | 0,02 |

Cette expérience montre que ces polymères sont capables d'associer un colorant insoluble dans l'eau.

### Exemple 4 : Adsorption de l'insuline

On prépare une solution aqueuse contenant 10 mg de polymère P2 par millilitre à pH 7,4 et 200 UI d'insuline (7,4 mg). On laisse incuber les solutions pendant deux heures à température ambiante et on sépare l'insuline libre de l'insuline associée par ultrafiltration (seuil à 100 KDa, 15 minutes sous 10 000 G à 18 °C). L'insuline libre récupérée dans le filtrat est ensuite dosée par CLHP (Chromatographie Liquide Haute Performance) et l'on déduit la quantité d'insuline associée. La quantité d'insuline associée est de 110 UI. En comparaison, la quantité d'insuline associée au polyglutamate de référence est nulle.

## Revendications

1. Polyaminoacide comprenant des unités aspartiques et/ou des unités glutamiques, dont certaines sont porteuses d'au moins un greffon, **caractérisé :**
• **en ce qu'**au moins un de ces greffons est relié à une unité aspartique ou glutamique, par l'intermédiaire d'une liaison amide,
• **en ce qu'**au moins une partie de ces greffons comprend un ou plusieurs (oligo)aminoacide(s), à l'exclusion des greffons porteurs d'au moins un diacide carboxylique cyclisable en anhydride,
• et **en ce que** l'unité ou les unités "acides aminés" comprises dans l'(oligo)aminoacide sont choisies parmi celles ayant un groupement alkyle ou aryle en alpha, de préférence parmi celles comprises dans le groupe comportant l'alanine, la valine, la leucine, l'isoleucine et la phénylalanine.

2. Polyaminoacide selon la revendication 1, **caractérisé en ce que** l'oligoaminoacide ou les (oligo)aminoacides est (sont) constitué(s) (chacun) par des unités "acide aminé" identiques entre-elles.

3. Polyaminoacide selon la revendication 1 ou 2, **caractérisé par** la formule générale (**I**) suivante : 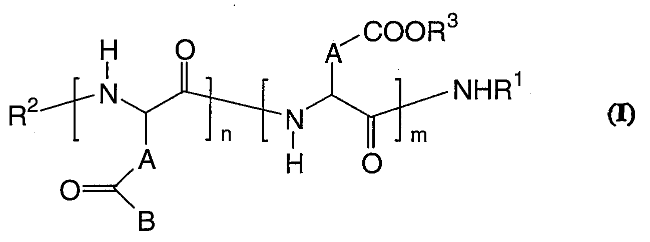 dans laquelle :
■ R¹ représente un H, un alkyle linéaire en C2 à C10 ou ramifié en C3 à C10, un benzyle, une unité "acide aminé" terminale;
■ R² représente un H, un groupe acyle linéaire en C2 à C10 ou ramifié en C3 à C10, ou un pyroglutamate
■ R³ est un H ou une entité cationique, de préférence sélectionnée dans le groupe comprenant :
- les cations métalliques avantageusement choisis dans le sous-groupe comprenant : le sodium, le potassium, le calcium, le magnésium,
- les cations organiques avantageusement choisis dans le sous-groupe comprenant :
• les cations à base d'amine,
• les cations à base d'oligoamine
• les cations à base de polyamine (la polyéthylèneimine étant particulièrement préférée),
• les cations à base d'acide(s) aminé(s) avantageusement choisis dans la classe comprenant les cations à base de lysine ou d'arginine,
- ou les polyaminoacides cationiques avantageusement choisis dans le sous-groupe comprenant la polylysine ou l'oligolysine;
■ les n groupements B représentent chacun indépendamment les uns des autres un radical monovalent de formule suivante : 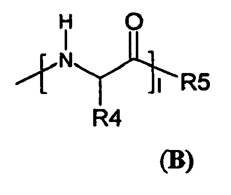
dans laquelle :
- R⁴ représente un méthyle(alanine), isopropyle (valine), isobutyle (leucine), secbutyle (isoleucine), benzyle (phenylalanine) ;
- R⁵ représente un groupement choisi parmi OH, NH₂, un groupement allcoxy en C1 à C5, un benzyloxy ;
■ A représente indépendamment un -CH₂- (unité aspartique) ou -CH₂-CH₂-(unité glutamique) ;
■ n/(n+m) est défini comme le taux de greffage molaire et varie de 0,5 à 100 % molaire;
■ n + m varie de 3 à 1000, de préférence entre 30 et 300 ;
■ 1 varie de 1 à 6.

4. Polyaminoacide selon la revendication 1, **caractérisé en ce que** tous les acides aminés constituant l'(oligo)aminoacide sont de types L.

5. Polyaminoacide selon la revendication 1, **caractérisé en ce qu'**il est constitué d'un homopolymère d'alpha-L-glutamate ou d'alpha-L-glutamique.

6. Polyaminoacide selon la revendication 1, **caractérisé en ce qu'**il est constitué d'un homopolymère d'alpha-L-aspartate ou d'alpha-L-aspartique.

7. Polyaminoacide selon la revendication 1, **caractérisé en ce qu'**il est constitué d'un copolymère d'alpha-L-aspartate/alpha-L-glutamate ou d'alpha-L-aspartique/alpha-L-glutamique.

8. Polyaminoacide selon la revendication 1, **caractérisé en ce que** la distribution des unités aspartiques et/ou glutamiques porteuses de greffons est telle que les polymères ainsi constitués sont soit aléatoires, soit de type bloc, soit de type multibloc.

9. Polyaminoacide selon la revendication 1, **caractérisés en ce que** sa masse molaire se situe entre 2 000 et 100 000 g/mole, et de préférence entre 5 000 et 40 000 g/mole.

10. Polyaminoacide selon la revendication 1, **caractérisé en ce que** le taux de greffage molaire se situe entre 2 et 70 %, et de préférence entre 5 et 40 %.

11. Composition pharmaceutique, cosmétique, diététique ou phytosanitaire comprenant au moins un polyaminoacide selon la revendication 1.

12. Composition selon la revendication 11, **caractérisée en ce qu'**elle comprend au moins un principe actif.

13. Composition, notamment selon la revendication 11, pharmaceutique, cosmétique, diététique ou phytosanitaire comprenant au moins un polyaminoacide comprenant des unités aspartiques et/ou des unités glutamiques dont certaines sont porteuses d'au moins un greffon :
• au moins un de ces greffons étant relié à une unité aspartique ou glutamique, par l'intermédiaire d'une liaison amide,
• au moins une partie de ces greffons comprenant un ou plusieurs (oligo)aminoacide(s),
• et les greffons porteurs d'au moins un diacide carboxylique cyclisable en anhydride étant exclus,
**caractérisée en ce qu'**elle comprend au moins un principe actif associé au(x) polyaminoacide(s) par une ou plusieurs liaisons autre(s) qu'une (ou que des) liaison(s) chimique(s) covalente(s).

14. Composition selon la revendication 12 ou 13, **caractérisée en ce que** le principe actif est une protéine, une glycoprotéine, une protéine liée à une ou plusieurs chaînes polyalkylèneglycol {de préférence PolyÉthylèneGlycol (PEG) : "protéine-PEGylée"}, un polysaccharide, un liposaccharide, un oligonucléotide, un polynucléotide ou un peptide.

15. Composition selon la revendication 12 ou 13, **caractérisée en ce que** le principe actif est une petite molécule organique hydrophobe, hydrophile ou amphiphile.

16. Composition selon la revendication 11, 12 ou 13, **caractérisée en ce qu'**elle peut être administrée par voie orale, parentérale, nasale, vaginale, oculaire, sous-cutanée, intraveineuse, intramusculaire, intradermique, intra péritonéale, intracérébrale ou buccale.

17. Composition selon la revendication 11, 12 ou 13, **caractérisée en ce qu'**elle est sous forme d'un gel, d'une émulsion, de micelles, de nanoparticules, de microparticules, d'une poudre ou d'un film.

18. Composition selon la revendication 11, 12 ou 13, **caractérisée en ce qu'**elle est une suspension colloïdale de nanoparticules et/ou de microparticules et/ou de micelles de polyaminoacides, dans une phase aqueuse.

19. Composition selon la revendication 11, 12 ou 13, **caractérisée en ce qu'**elle est sous forme de solution dans un solvant biocompatible et **en ce qu'**elle peut être injectée par voie en sous-cutanée, intramusculaire ou dans une tumeur.

20. Composition selon la revendication 11, 12 ou 13, **caractérisée en ce qu'**elle est apte à former un dépôt sur le site d'injection.

21. Procédé de préparation:
• de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyÉthylèneGlycol (PEG), on parle alors de protéines "PEGylées"}, des peptides, des polysaccharides, des liposaccharides, des oligonucléotides, des polynucléotides et des petites molécules organiques hydrophobes, hydrophiles ou amphiphiles ;
• et/ou des nutriments ;
• et/ou de produits cosmétiques ou phytosanitaires ;
**caractérisé en ce qu'**il consiste essentiellement à mettre en oeuvre au moins un polyaminoacide selon la revendication 1 et/ou la composition selon la revendication 11, 12 ou 13.

## Claims

1. Polyamino acid comprising aspartic units and/or glutamic units, some of which bear at least one graft, **characterized:**
• **in that** at least one of these grafts is linked to an aspartic or glutamic unit via an amide bond;
• **in that** at least some of these grafts comprise one or more (oligo)amino acid(s), with the exclusion of grafts bearing at least one dicarboxylic acid that can be cyclized to an anhydride; and
• **in that** the "amino acid" unit or units included in the (oligo)amino acid are chosen from those having an alkyl or aryle group in the alpha position, preferably from those in the group comprising alanine, valine, leucine, isoleucine and phenylalanine.

2. Polyamino acid according to Claim 1, **characterized in that** the oligoamino acid or (oligo)amino acids are (each) composed of "amino acid" units that are identical to each other.

3. Polyamino acid according to Claim 1 or 2, **characterized by** the general formula (I) below: 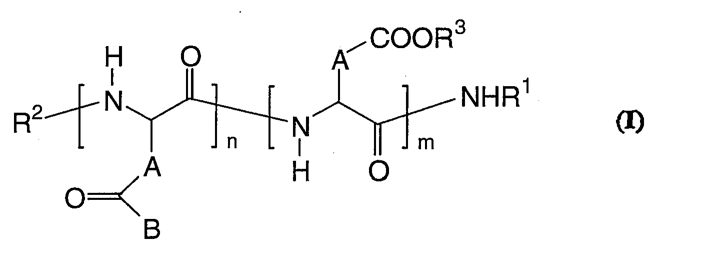 in which:
■ R¹ represents H, a C₂ to C₁₀ linear or C₃ to C₁₀ branched alkyl group, a benzyl group or a terminal "amino acid" unit;
■ R² represents H, a C₂ to C₁₀ linear or C₃ to C₁₀ branched acyl group or a pyroglutamate;
■ R³ is H or a cationic species, preferably chosen from the group comprising:
- metal cations advantageously chosen from the subgroup comprising: sodium, potassium, calcium or magnesium;
- organic cations advantageously chosen from the subgroup comprising:
• amine-based cations;
• oligoamine-based cations;
• cations based on polyamine (polyethyleneimine being particularly preferred);
• cations based on amino acid(s) advantageously chosen from the class comprising cations based on lysine or arginine;
- or cationic polyamino acids advantageously chosen from the subgroup comprising polylysine or oligolysine;
■ the n groups B each representing independently of one another a monovalent radical of the following formula: 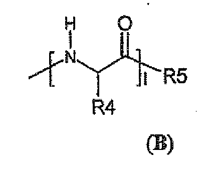
in which:
- R⁴ represents a methyl (alanine), isopropyl (valine), isobutyl (leucine), *sec*-butyl (isoleucine) or benzyl (phenylalanine) group;
- R⁵ represents a group chosen from OH, NH₂, a C₁ to C₅ alkoxy group or a benzyloxy group;
■ A independently represents a -CH₂- (aspartic unit) or -CH₂-CH₂- (glutamic unit) group;
■ n/(n+m) is defined as the molar degree of grafting and varies from 0.5 to 100 mol%;
■ n + m varies from 3 to 1000, preferably between 30 and 300; and
■ l varies from 1 to 6.

4. Polyamino acid according to Claim 1, **characterized in that** all the amino acids forming the (oligo)amino acid are of L types.

5. Polyamino acid according to Claim 1, **characterized in that** it is composed of an α-L-glutamate or α-L-glutamic homopolymer.

6. Polyamino acid according to Claim 1, **characterized in that** it is composed of an α-L-aspartate or α-L-aspartic homopolymer.

7. Polyamino acid according to Claim 1, **characterized in that** it is composed of an α-L-aspartate/α-L-glutamate or α-L-aspartic/α-L-glutamic copolymer.

8. Polyamino acid according to Claim 1, **characterized in that** the distribution of aspartic and/or glutamic units bearing grafts is such that the polymers thus formed are either random, or of block type, or of multiblock type.

9. Polyamino acid according to Claim 1, **characterized in that** its molecular weight lies between 2000 and 100 000 g/mol, and preferably between 5000 and 40 000 g/mol.

10. Polyamino acid according to Claim 1, **characterized in that** the molar degree of grafting lies between 2 and 70%, and preferably between 5 and 40%.

11. Pharmaceutical, cosmetic, dietetic or plant-protection composition comprising at least one polyamino acid according to Claim 1.

12. Composition according to Claim 11, **characterized in that** it comprises at least one active principle.

13. Pharmaceutical, cosmetic, dietetic or plant-protection composition, especially according to Claim 11, comprising at least one polyamino acid comprising aspartic units and/or glutamic units, some of which bear at least one graft:
• at least one of these grafts being linked to an aspartic or glutamic unit, via an amide bond;
• at least some of these grafts comprising one or more (oligo)amino acid(s); and
• the grafts bearing at least one dicarboxylic acid that can be cyclized to an anhydride being excluded,
**characterized in that** it comprises at least one active principle joined to the polyamino acid(s) by one or more bonds other than a (or some) covalent chemical bond(s).

14. Composition according to Claim 12 or 13, **characterized in that** the active principle is a protein, a glycoprotein, a protein linked to one or more polyalkylene glycol chains {preferably polyethylene glycol (PEG): "PEGylated protein"}, a polysaccharide, a liposaccharide, an oligonucleotide, a polynucleotide or a peptide.

15. Composition according to Claim 12 or 13, **characterized in that** the active principle is a small hydrophobic, hydrophilic or amphiphilic organic molecule.

16. Composition according to Claim 11, 12 or 13, **characterized in that** it may be administered via the oral, parenteral, nasal, vaginal, ocular, subcutaneous, intravenous, intramuscular, intradermal, intraperitoneal, intracerebral or buccal route.

17. Composition according to Claim 11, 12 or 13, **characterized in that** it is in the form of a gel, an emulsion, micelles, nanoparticles, microparticles, a powder or a film.

18. Composition according to Claim 11, 12 or 13, **characterized in that** it is a colloidal suspension of nanoparticles and/or of microparticles and/or of micelles of polyamino acids, in an aqueous phase.

19. Composition according to Claim 11, 12 or 13, **characterized in that** it is in the form of a solution in a biocompatible solvent and **in that** it may be injected subcutaneously, intramuscularly or into a tumour.

20. Composition according to Claim 11, 12 or 13, **characterized in that** it is capable of forming a deposit at the injection site.

21. Method for preparing:
• medications, in particular for oral, nasal, vaginal, ocular, subcutaneous, intravenous, intramuscular, intradermal, intraperitoneal or intracerebral administration, the active principles of these medications possibly being, especially, proteins, glycoproteins, proteins linked to one or more polyalkylene glycol chains {for example polyethylene glycol (PEG), which are then referred to as "PEGylated" proteins}, peptides, polysaccharides, liposaccharides, oligonucleotides, polynucleotides and small hydrophobic, hydrophilic or amphiphilic organic molecules; and/or
• nutrients; and/or
• cosmetic or plant-protection products,
**characterized in that** it mainly consists in using at least one polyamino acid according to Claim 1 and/or the composition according to Claim 11, 12 or 13.

## Patentansprüche

1. Polyaminosäure, umfassend Asparaginsäure und/oder Glutaminsäure, von denen einige Träger mindestens eines aufgepfropften Rests sind, **dadurch gekennzeichnet, daß**
• mindestens eine dieser Pfropfungen mit einer Asparagin- oder Glutaminsäure-Einheit durch eine Amidbindung verbunden ist,
• mindestens ein Teil dieser aufgepfropften Reste eine oder mehrere (Oligo)aminosäure(n) umfaßt, außer aufgepfropften Resten, die mindestens eine in ein Anhydrid cyclisierbare Dicarbonsäure sind, und
• die Einheit oder die "Aminosäure"-Einheiten, die von der (Oligo)aminosäure umfaßt werden, aus solchen ausgewählt sind, die eine Alkyl- oder Arylgruppe an der Alphaposition haben, vorzugsweise aus solchen, die von der Gruppe umfaßt werden, die Alanin, Valin, Leucin, Isoleucin und Phenylalanin umfaßt.

2. Polyaminosäure gemäß Anspruch 1, **dadurch gekennzeichnet, daß** die Oligoaminosäure oder die (Oligo)aminosäuren (jeweils) aus untereinander identischen "Aminosäure"-Einheiten gebildet ist/sind.

3. Polyaminosäure gemäß Anspruch 1 oder 2, **gekennzeichnet durch** die folgende allgemeine Formel (I): 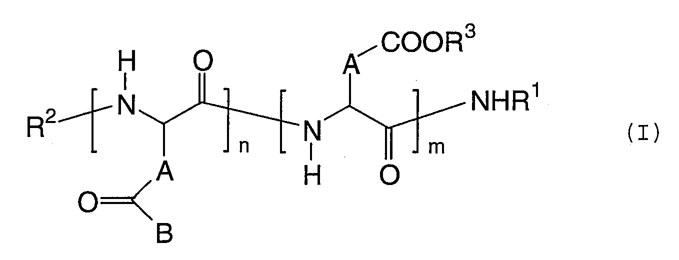 in der
R¹ H, ein lineares C₂₋₁₀-Alkyl oder verzweigtes C₃₋₁₀-Alkyl, ein Benzyl, eine terminale "Aminosäure"-Einheit darstellt;
■ R² H, eine lineare C₂₋₁₀-Acylgruppe oder eine verzweigte C₃₋₁₀-Acylgruppe oder ein Pyroglutamat darstellt;
■ R³ H oder ein kationischer Rest ist, vorzugsweise ausgewählt aus der Gruppe umfassend:
- metallische Kationen, vorzugsweise ausgewählt aus der Untergruppe umfassend: Natrium, Kalium, Calcium, Magnesium,
- organische Kationen, vorzugsweise ausgewählt aus der Untergruppe umfassend
• Kationen auf Aminbasis,
• Kationen auf Oligoaminbasis,
• Kationen auf Polyaminbasis (Polyethylenimin ist besonders bevorzugt)
• Kationen auf Aminosäure(n)-Basis, vorzugsweise ausgewählt aus der Klasse, die Kationen auf Grundlage von Lysin oder Arginin umfaßt,
- kationische Polyaminosäuren, die vorzugsweise ausgewählt sind aus der Untergruppe, die Polylysin oder Oligolysin umfaßt;
■ die n B-Gruppen stellen jeweils unabhängig voneinander einen monovalenten Rest der folgenden Formel dar: 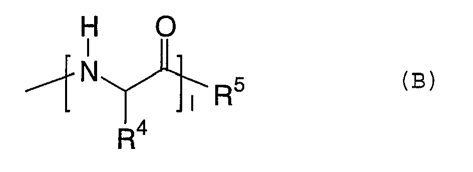 in der
- R⁴ Methyl(alanin), Isopropyl(valin), Isobutyl(leucin), Secbutyl(isoleucin), Benzyl(phenylalanin) darstellt;
- R⁵ eine Gruppe darstellt, die ausgewählt ist aus OH, NH₂, einer C₁₋₅-Alkyoxygruppe, einem Benzyloxy;
■ A unabhängig eine -CH₂- (Asparaginsäure-Einheit) oder -CH2-CH₂- (Glutaminsäure-Einheit) darstellt;
■ n/(n+m) wird als Ausmaß der molaren aufgepfropften Reste definiert und variiert von 0,5 bis 100 mol%;
■ n + m variiert zwischen 3 und 1.000, vorzugsweise zwischen 30 und 300;
■ 1 variiert zwischen 1 und 6.

4. Polyaminosäure gemäß Anspruch 1, **dadurch gekennzeichnet, daß** alle Aminosäuren, die die (Oligo)aminosäure bilden, vom L-Typ sind.

5. Polyaminosäure gemäß Anspruch 1, **dadurch gekennzeichnet, daß** sie aus einem alpha-L-Glutamat- oder alpha-L-Glutaminsäure-Homopolymer gebildet ist.

6. Polyaminosäure gemäß Anspruch 1, **dadurch gekennzeichnet, daß** sie aus einem alpha-L-Asparat- oder alpha-L-Asparaginsäure-Homopolymer gebildet ist.

7. Polyaminosäure gemäß Anspruch 1, **dadurch gekennzeichnet, daß** sie aus einem alpha-L-Aspartat/alpha-L-Glutamat- oder alpha-L-Asparaginsäure/alpha-L-Glutaminsäure-Copolymer gebildet ist.

8. Polyaminosäure gemäß Anspruch 1, **dadurch gekennzeichnet, daß** die Verteilung der Asparaginsäure- und/oder Glutaminsäure-Einheiten, die Träger der aufgepfropften Reste sind, so ist, daß die **dadurch** gebildeten Polymere entweder statistisch oder vom Blocktyp oder vom Multiblocktyp sind.

9. Polyaminosäure gemäß Anspruch 1, **dadurch gekennzeichnet, daß** ihre molare Masse zwischen 2.000 und 100.000 g/mol liegt und vorzugsweise zwischen 5.000 und 40.000 g/mol.

10. Polyaminosäure gemäß Anspruch 1, **dadurch gekennzeichnet, daß** das Ausmaß der molaren aufgepfropften Reste zwischen 2 und 70 % und vorzugsweise zwischen 5 und 40 % liegt.

11. Pharmazeutische, kosmetische, diätetische oder Pflanzenschutzzusammensetzung, umfassend mindestens eine Polyaminosäure gemäß Anspruch 1.

12. Zusammensetzung gemäß Anspruch 11, **dadurch gekennzeichnet, daß** sie mindestens einen Wirkstoff umfaßt.

13. Pharmazeutische, kosmetische, diätetische oder Pflanzenschutzzusammensetzung, insbesondere gemäß Anspruch 11, umfassend mindestens eine Polyaminosäure umfassend Asparaginsäure- und/oder Glutaminsäure-Einheiten, von denen einige Träger mindestens eines aufgepfropften Rests sind:
• mindestens einer dieser aufgepfropften Reste mit einer Asparagin- oder Glutaminsäure-Einheit durch eine Amidbindung verbunden ist,
• mindestens ein Teil dieser aufgepfropften Reste einen oder mehrere (Oligo)aminosäuren umfaßt,
• außer Pfropfungen, die mindestens eine in ein Anhydrid cyclisierbare Dicarbonsäure sind,
**dadurch gekennzeichnet, daß** sie mindestens einen Wirkstoff umfaßt, der mit (der) Polyaminosäure(n) durch eine oder mehrere andere Verbindung(en) als (oder durch) chemisch kovalenten Verbindungen verbunden ist.

14. Zusammensetzung gemäß Anspruch 12 oder 13, **dadurch gekennzeichnet, daß** der Wirkstoff ein Protein, ein Glycoprotein, ein mit einer oder mehreren Polyalkylenglycolketten verbundenes Protein (vorzugsweise Polyethylenglycol (PEG): "PEGyliniertes" Protein), ein Polysaccharid, ein Liposaccharid, ein Oligonukleotid, ein Polynukleotid oder ein Peptid ist.

15. Zusammensetzung gemäß Anspruch 12 oder 13, **dadurch gekennzeichnet, daß** der Wirkstoff ein kleines hydrophobes, hydrophiles oder amphiphiles organisches Molekül ist.

16. Zusammensetzung gemäß Anspruch 11, 12 oder 13, **dadurch gekennzeichnet, daß** sie auf oralem, parenteralem, nasalem, vaginalem, okularem, subkutanem, intravenösem, intramuskulärem, intradermalem, intraperitonealem, intrazerebralem oder bukkalem Weg verabreicht werden kann.

17. Zusammensetzung gemäß Anspruch 11, 12 oder 13, **dadurch gekennzeichnet, daß** sie in Form eines Gels, einer Emulsion, von Mizellen, Nanopartikeln, Mikropartikeln, einem Pulver oder einem Film vorliegt.

18. Zusammensetzung gemäß Anspruch 11, 12 oder 13, **dadurch gekennzeichnet, daß** sie eine kolloidale Suspension aus Nanopartikeln und/oder Mikropartikeln und/oder Polyaminosäure-Mizellen in einer wäßrigen Phase ist.

19. Zusammensetzung gemäß Anspruch 11, 12 oder 13, **dadurch gekennzeichnet, daß** sie in Form einer Lösung in einem biokompatiblen Lösungsmittel vorliegt, und daß sie auf subkutanem, intramuskulärem Wege oder in einen Tumor injiziert werden kann.

20. Zusammensetzung gemäß Anspruch 11, 12 oder 13, **dadurch gekennzeichnet, daß** sie in der Lage ist, an der Injektionsstelle ein Depot zu bilden.

21. Verfahren zur Herstellung von
• Medikamenten, insbesondere zur oralen, nasalen, vaginalen, okularen, subkutanen, intravenösen, intramuskulären, intradermalen, intraperitonealen oder intrazerebralen Verabreichung, wobei die Wirkstoffe dieser Medikamente insbesondere Proteine, Glycoproteine, Proteine, die mit einer oder mehreren Polyalkylenglycolketten verbunden sind (z.B. Polyethylenglycol (PEG), man spricht dann von "PEGylinierten" Proteinen"), Peptide, Polysaccharide, Liposaccharide, Oligonukleotide, Polynukleotide und kleine hydrophobe, hydrophile oder amphiphile organische Moleküle sein können;
• und/oder Nährstoffen;
• und/oder kosmetischen oder Pflanzenschutzprodukten;
**dadurch gekennzeichnet, daß** es im wesentlichen aus der Herstellung mindestens einer Polyaminosäure gemäß Anspruch 1 und/oder der Zusammensetzung gemäß Ansprüchen 11, 12 oder 13 besteht.
